# EUROPÄISCHE PATENTSCHRIFT

(11) **EP 2 280 920 B1**
(45) Veröffentlichungstag und Bekanntmachung des Hinweises auf die Patenterteilung: **28.12.2011**
(21) Anmeldenummer: 09757368.7
(22) Anmeldetag: 28.04.2009
(51) Int. Cl.: C07C 45/50, B01J 31/02, B01J 31/18

(54) **VERFAHREN ZUR HERSTELLUNG VON C5-ALDEHYDGEMISCHEN MIT HOHEM N-PENTANALANTEIL**
METHOD FOR PRODUCING C5-ALDEHYDE MIXTURES HAVING A HIGH N-PENTANAL CONTENT
PROCÉDÉ POUR PRODUIRE DES MÉLANGES D' ALDÉHYDES C5 FORTE TENEUR EN PENTANAL

(30) Priorität: 03.06.2008 DE 102008002187
(43) Veröffentlichungstag der Anmeldung: 09.02.2011
(73) Patentinhaber: Evonik Oxeno GmbH, 45772 Marl (DE)
(72) Erfinder: LÜKEN, Hans-Gerd, 45770 Marl (DE); FRIDAG, Dirk, 45721 Haltern am See (DE); LENZ, Udo, 45657 Recklinghausen (DE); SCHULTE-ALTHOFF, Hermann-Josef, 45721 Haltern am See (DE); WIESE, Klaus-Diether, 45721 Haltern am See (DE); KAIZIK, Alfred, 45772 Marl (DE); MUHLACK, Patrick, 45659 Recklinghausen (DE); BÜSCHKEN, Wilfried, 45721 Haltern am See (DE); HÖPER, Frank, 45721 Haltern am See (DE)
(86) Internationale Anmeldenummer: PCT/EP2009/055133
(87) Internationale Veröffentlichungsnummer: WO 2009/146985

(56) Entgegenhaltungen:
- EP-A- 1 201 675
- WO-A-2007/028660
- WO-A-2008/124468
- US-A- 4 668 651
- US-A1- 2004 138 508

## Beschreibung

Die vorliegende Erfindung betrifft ein Verfahren zur Herstellung von C₅-Aldehydgemischen mit hohem n-Pentanalanteil aus einem lineare Butene enthaltenden Kohlenwasserstoffgemisch. Insbesondere betrifft die Erfindung die Herstellung von C₅-Aldehydgemischen mit einem Verhältnis von n-Pentanal zu 2-Methylbutanal von größer als 90 zu 10 aus einem lineare Butene enthaltenden Kohlenwasserstoffgemisch.

C₅-Aldehyde sind Ausgangsstoffe zur Erzeugung von Pentanolen Pentansäuren, und Pentylaminen. Durch Aldolkondensation und Totalhydrierung des Aldolkondensats können aus ihnen Decanole gewonnen werden, die Zwischenprodukte für die Herstellung von Weichmachern, Detergenzien und Schmiermitteln sind. Durch ihre Aldolkondensation, Hydrierung der olefinischen Doppelbindung des Aldolkondensats und anschließende Oxidation der aldehydischen Gruppe können Decansäuren erhalten werden, die beispielsweise zur Herstellung von Schmiermitteln oder Detergenzien verwendet werden können. In diesem Einsatzbereich ist es wichtig, dass die C₅-Aldehyde möglichst ausschließlich aus der linearen Verbindung n-Pentanal bestehen bzw. der Anteil an verzweigten C₅-Aldehyden, wie insbesondere 2-Methylbutanal, möglichst gering ist.

C₅-Aldehyde können durch Hydroformylierung von ungesättigten C₄-Verbindungen erhalten werden. Entsprechende industriell verfügbare Ausgangsstoffe sind Kohlenwasserstoffgemische, die 1-Buten, die 2-Butene (Z und E) und Isobuten enthalten. Entsprechend der Stellung der C-C-Doppelbindung in den ungesättigten C₄-Verbindungen und in Abhängigkeit der Reaktionsbedingungen liefert deren Hydroformylierung lineare und verzweigte C₅-Aldehyde bzw. C₅-Aldehydgemische in unterschiedlicher Selektivität.

1-Buten kann in über 90%iger Selektivität zu n-Pentanal hydroformyliert werden. Als Katalysatoren werden meistens Komplexe aus Rhodium und Monophosphinen dazu verwendet. Ein Standardkatalysator ist beispielsweise ein Komplex, bestehend aus Rhodium und Triphenylphosphin. Die Umsetzung kann in homogener Phase, wie etwa in EP 0 562 451 beschrieben, oder in heterogener Phase, wie etwa in DE 026 27 354 oder EP 0 562 451 beschrieben, durchgeführt werden.

Optional kann die Hydroformylierung der 1-Olefine im Mehrphasensystem, wobei Edukt, Produkt und Synthesegas in einer kontinuierlichen Katalysatorphase dispergiert sind, unter hohen Leerrohrgeschwindigkeiten durchgeführt werden. Solche Verfahren werden beispielsweise in DE 199 25 384 A1 und DE 199 57 528 A1 beschrieben.

Die selektive Herstellung von n-Pentanal aus 2-Butenen oder von Gemischen davon ist wesentlich schwieriger. In DE 101 08 474, DE 101 084 75, DE 101 084 76 und DE 102 252 82 wird die Herstellung von C₅-Aldehydgemischen durch Hydroformylierung eines Gemisches linearer Butene beschrieben. Die technischen Lehren aller dieser Schriften haben gemeinsam, dass in mindestens einem Hydroformylierungsschritt ein Rhodiumkatalysator mit einem Diphosphinliganden, der ein Xanthengerüst aufweist, verwendet wird. Mit diesem Katalysator können 2-Butene unter isomerisierenden Bedingungen hydroformyliert werden. Das Verhältnis von n-Pentanal zu 2-Methylbutanal liegt bei bestenfalls 85 zu 15. Die Schriften DE 101 08 474 und DE 101 08 475 beschreiben Verfahren, bei denen die Hydroformylierung zweistufig erfolgt. In der ersten Hydroformylierungsstufe wird unter Verwendung eines Katalysators, bestehend aus Rhodium und einem Monophosphin als Ligand, 1-Buten in einer Selektivität von 90 % zu n-Pentanal umgesetzt. Die nicht umgesetzten Butene, hauptsächlich 2-Butene, werden in der zweiten Hydroformylierungsstufe unter Verwendung des oben genannten Rhodium/Bisphosphin umgesetzt. Die Schriften DE 101 08 476 und DE 102 25 282 beschreiben einstufige Hydroformylierungsverfahren.
Höhere Selektivitäten an n-Pentanal bei der Hydroformylierung von 2-Butenen können bei Verwendung eines Katalysators, bestehend aus Rhodium und sperrigen aromatischen Bisphosphiten erhalten werden, wie sie beispielsweise in EP 0 213 639 beschrieben werden. Allerdings nimmt die Selektivität mit der Zeit stark ab.

In DE 10 2005 042464 werden für die Hydroformylierung von Olefinen Katalysatorsysteme genannt, die einen Komplex, bestehend aus Rhodium und einer phospororganischen Verbindung sowie ein sterisch gehindertes sekundäres Amin enthalten. Diese Katalysatorsysteme zeichnen sich durch hohe Langzeitstabilität aus. Sie können für die Hydroformylierung von Olefinen mit 3 bis 16 C-Atomen eingesetzt werden. In den Beispielen wurde nur 1-Octen hydroformyliert. Es entstanden Gemische mehrerer C₉-Aldehyde, über deren Isomerenverteilung aber nichts ausgesagt ist.

Der vorliegenden Erfindung lag daher die Aufgabe zugrunde, ein Verfahren anzugeben, mit dem aus einem lineare Butene enthaltenden Kohlenwasserstoffgemisch n-Pentanal in möglichst hoher Selektivität erhalten werden kann.

Es wurde nun gefunden, dass ein spezielles Katalysatorsystem, bestehend aus Rhodium und einem organischen Bisphosphit sowie aus einem sterisch gehinderten sekundären Amin, eine selektive Hydroformylierung von 2-Butenen zu n-Pentanal bewirkt und damit in einem Verfahren zur Herstellung von C₅-Aldehydgemischen mit hohem n-Pentanalanteil aus einem lineare Butene enthaltenden Kohlenwasserstoffgemisch besonders gut geeignet ist. Das Katalysatorsystem ist eine komplexe Rhodiumverbindung, die mindestens einen Liganden der Formel **I** 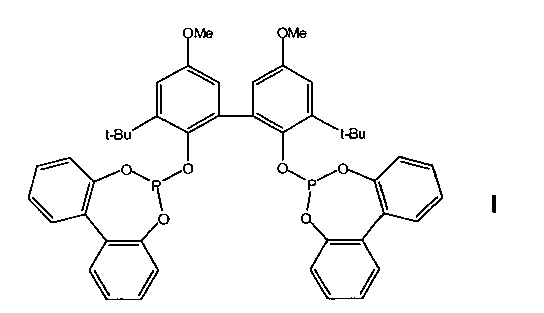 und mindestens ein sekundäres sterisch gehindertes sekundäres Amine der allgemeinen Formel II, 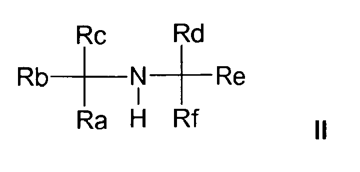 worin Ra, Rb, Rc, Rd, Re und Rf gleiche oder unterschiedliche Kohlenwasserstoffreste die auch untereinander verbunden sein können, aufweist.

Gegenstand der vorliegenden Erfindung ist ein Verfahren zur Herstellung von C₅-Aldehydgemischen aus einem lineare Butene enthaltenden Kohlenwasserstoffgemisch durch endständige Hydroformylierung unter isomerisierenden Bedingungen, wobei ein Katalysatorsystem eingesetzt wird, enthaltend Rhodium, ein Bisphosphit-Ligand der Formel I und einem Amin der Formel II.

Das Katalysatorsystem hat den Vorteil, dass es geeignet ist, 2-Butene oder Gemische linearer Butene mit beliebigen Verhältnis von 2-Butenen zu 1-Buten in einer Selektivität von über 90 % zu n-Pentanal zu hydroformylieren. Zudem ist der Verlust an Rhodium und Bisphosphit so gering, dass im Vergleich zu anderen Katalysatorsystemen ein wirtschaftlicher Vorteil besteht. Darüber hinaus ist es vorteilhaft, dass die Butenhydroformylierung mit diesem Katalysatorsystem in bestehenden Anlagen für die Propenhydroformylierung durchgeführt werden kann.

### Einsatzstoffe

Einsatzstoffe für das erfindungsgemäße Verfahren sind lineare Butene und beliebige Mischungen davon. Diese Gemische können bis zu 5 Massen-% Isobuten enthalten bezogen auf die C₄-Olefinfraktion. Bevorzugt werden Gemische eingesetzt, die weniger als 1 Massen-% Isobuten, insbesondere weniger als 0,1 Massen-% Isobuten enthalten. In den Einsatzstoffgemischen können gesättigte Kohlenwasserstoffe mit 1 bis 7 C-Atomen, insbesondere mit 4 C-Atomen sowie Benzol und Toluol vorhanden sein. Bevorzugt werden C₄-Kohlenwasserstoffgemische verwendet, insbesondere solche, bei denen das Massenverhältnis aus der Summe der beiden 2-Butene zu 1-Buten größer als 2 zu 1, vorzugsweise größer als 5 zu 1 und ganz besonders bevorzugt größer als 10 zu 1 ist.

Einsatzstoffe für das erfindungsgemäße Verfahren können Gemische sein, die bei der Dehydrierung von n-Butan anfallen und aus denen Nebenprodukte und mehrfach ungesättigte Verbindungen abgetrennt worden sind.

Andere Einsatzstoffe sind Isobuten-arme Teilmengen, die bei der Aufarbeitung von C₄-Schnitten aus Hydrocrackern, FC-Crackern oder Steamcrackern anfallen. Ein Beispiel dafür ist das sogenannte Raffinat II, das aus einem C₄-Schnitt durch Entfernen der mehrfach ungesättigten Verbindungen, wie hauptsächlich Butadien, und Isobuten gewonnen wird. Es enthält 1-Buten, die beiden 2-Butene, n-Butan und Isobutan. Werden Isobutan und ein Teil des 1-Butens abgetrennt, erhält man Raffinat III. Raffinat II und Raffinat III sind geeignete Einsatzstoffe. Raffinat II und Raffinat III können für die Herstellung von Butenoligomeren genutzt werden. Die verbleibenden Restströme, bestehend aus n-Butan, linearen Butenen, hauptsächlich 2-Butenen und gegebenenfalls Isobutan, können ebenfalls im erfindungsgemäßen Verfahren eingesetzt werden.

Das eingesetzte Synthesegas hat ein molares Verhältnis von Wasserstoff zu Kohlenmonoxid im Bereich von 2 zu 1 bis 1 zu 2, insbesondere eines im Bereich von 1,1 zu 0,9 bis 0,9 zu 1,1.
Das verwendete Synthesegas wird vorzugsweise vor seinem Einsatz nach an sich bekannten Verfahren gereinigt. Es enthält vorzugsweise weniger als 1 Massen-ppm Schwefelverbindungen (gerechnet als elementarer Schwefel) und weniger als 1 Massen-ppm Sauerstoff. Optional kann das Synthesegas unter Reaktionsbedingungen inerte Gase, wie beispielsweise Methan oder Stickstoff, enthalten.

### Katalysatorsystem

In dem erfindungsgemäßen Verfahren wird ein Katalysatorsystem verwendet, das aus einer komplexen Rhodiumverbindung, die mindestens einen Liganden der Formel **I** aufweist, und mindestens aus einem sterisch gehinderten sekundärem Amin besteht.

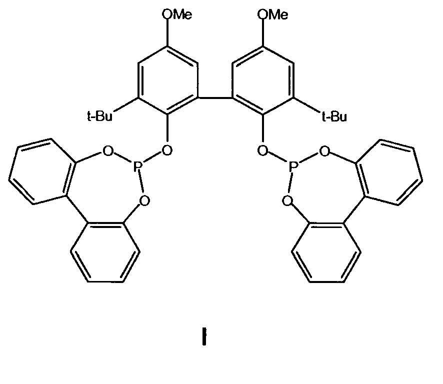

Als sterisch gehinderte sekundäre Amine werden Verbindungen mit der allgemeinen Formel II eingesetzt, 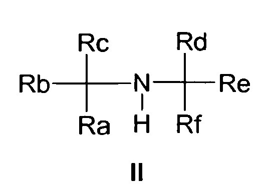 wobei Ra, Rb, Rc, Rd, Re und Rf gleiche oder unterschiedliche Kohlenwasserstoffreste die auch untereinander verbunden sein können, sind.

Bevorzugt werden im erfindungsgemäßen Verfahren sekundäre Amine eingesetzt, die eine 2,2,6,6-Tetramethylpiperidineinheit **IIa** 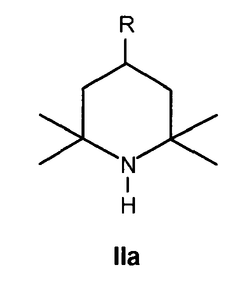 aufweisen, mit R gleich H, wie das 2,2,6,6-Tetramethylpiperidin selbst, einem organischen Rest, einer Hydroxylgruppe oder ein Halogen.
Der organische Rest R kann auch ein über ein Heteroatom, beispielsweise ein Sauerstoffatom, an die 2,2,6,6-Tetramethylpiperidin-Struktureinheit gebundener, organischer Rest sein. Insbesondere kann der organische Rest polymere Strukturen aufweisen oder ein 1 bis 50 Kohlenstoffatome und gegebenenfalls Heteroatome aufweisender organischer Rest sein. Besonders bevorzugt weist der organische Rest Carbonylgruppen, wie Keto-, Ester- oder Säureamid-Gruppen auf. Der organische, gegebenenfalls Heteroatome aufweisende Rest kann insbesondere ein substituierter oder unsubstituierter, aliphatischer, alicyclischer, aliphatisch-alicyclischer, heterocyclischer, aliphatisch-heterocyclischer, aromatischer, aromatischaromatischer oder aliphatisch-aromatischer Kohlenwasserstoffrest mit 1 bis 50 Kohlenstoffatomen sein, wobei die substituierten Kohlenwasserstoffreste Substituenten, ausgewählt aus primären, sekundären oder tertiären Alkylgruppen, alicyclischen Gruppen, aromatischen Gruppen, -N(R¹)₂, -NHR¹, -NH₂, Fluor, Chlor, Brom, Jod, -CN, -C(O)-R¹, -C(O)H oder -C(O)O-R¹, -CF₃, -O-R¹, -C(O)N-R¹, -OC(O)-R¹ und/oder -Si(R¹)₃, mit R¹ gleich einem monovalenten, bevorzugt 1 bis 20 Kohlenstoffatome aufweisenden Kohlenwasserstoffrest, aufweisen können. Sind mehrere Kohlenwasserstoffreste R¹ vorhanden, so können diese gleich oder unterschiedlich sein. Die Substituenten sind vorzugsweise beschränkt auf solche, die keinen Einfluss auf die Reaktion selbst haben. Besonders bevorzugte Substituenten können ausgewählt sein aus den Halogenen, wie z. B. Chlor, Brom oder Jod, den Alkylresten, wie z. B. Methyl, Ethyl, Propyl, iso-Propyl, Butyl, sec-Butyl, t-Butyl, neo-Pentyl, sec-Amyl, t-Amyl, iso-Octyl, t-Octyl, 2-Ethylhexyl, iso-Nonyl, iso-Decyl oder Octadecyl, den Arylresten, wie z. B. Phenyl, Naphthyl oder Anthracyl, den Alkylarylresten, wie z. B. Tolyl, Xylyl, Dimethylphenyl, Diethylphenyl, Trimethylphenyl, Triethylphenyl oder p-Alkylphenyl, den Aralkylresten, wie z. B. Benzyl oder Phenylethyl, den alicyclischen Resten, wie z. B. Cyclopentyl, Cyclohexyl, Cyclooctyl, Cyclohexylethyl oder 1-Methylcyclohexyl, den Alkoxyresten, wie z. B. Methoxy, Ethoxy, Propoxy, Butoxy oder Pentoxy, den Aryloxyresten, wie z. B. Phenoxy oder Naphthoxy, -OC(O)R¹ oder -C(O)R¹, wie z. B. Acetyl, Propionyl, Trimethylacetoxy, Triethylacetoxy oder Triphenylacetoxy, und den drei Kohlenwasserstoffreste aufweisenden Silylresten -Si(R¹)₃, wie z. B. Trimethylsilyl, Triethylsilyl oder Triphenylsilyl. Besonders bevorzugt sind Verbindungen der Formel IIa, die als Reste R solche aufweisen, die einen 2,2,6,6,-Tetramethylpiperidin-Rest und gegebenenfalls eine weitere -N(R¹)₂, -NHR¹ und/oder -NH₂ Gruppe enthalten.

Als sekundäre Amine, die eine Struktureinheit gemäß Formel **II** aufweisen, können ganz besonders bevorzugt die nachfolgend aufgeführten Verbindungen mit den Strukturformeln **IIb** bis **IIg** oder deren Derivate eingesetzt werden. 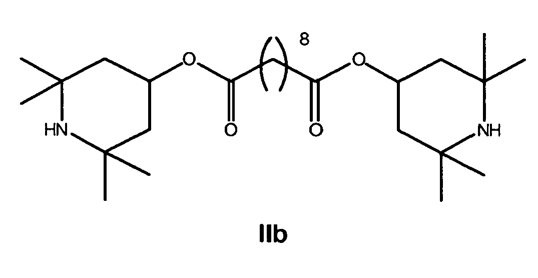 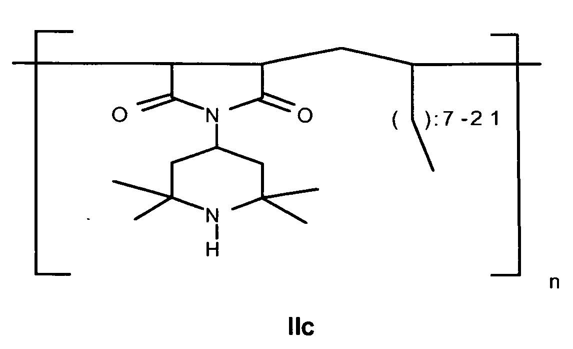 mit n = 1 bis 20, vorzugsweise 1 bis 10 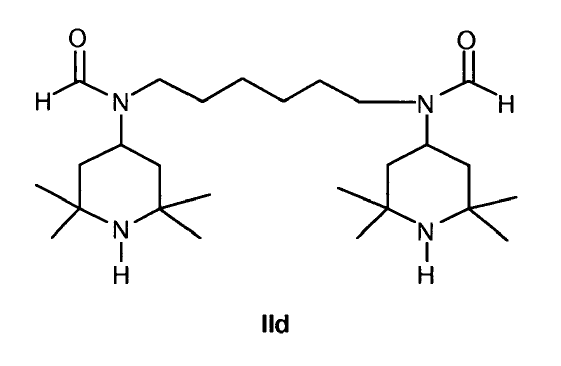 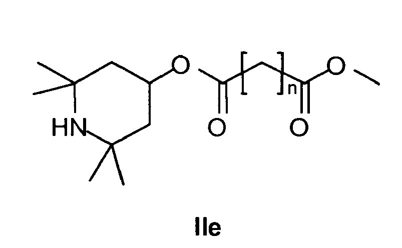 mit n = 1 bis 12, vorzugsweise 8 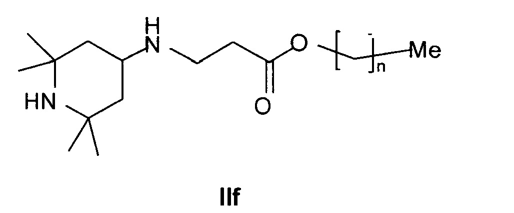 mit n = 1 bis 17, vorzugsweise 13

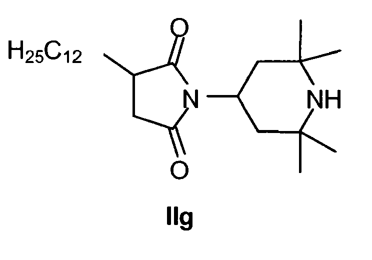

Es können auch Gemische, enthaltend zwei oder mehrere sterisch gehinderte Amine, eingesetzt werden.
Insbesondere wird im erfindungsgemäßen Verfahren das Amin mit der Formel **IIb**, Sebacinsäuredi-4(2,2,6,6-tetramethylpiperidinyl)ester bevorzugt eingesetzt.

Im Katalysatorsystem liegen die drei Komponenten, Rhodium, Bisphosphit der Formel **I** und sterisch gehindertes Amin **II**, in bestimmten molaren Verhältnissen vor.

Das molare Verhältnis von Rhodium zu Bisphosphit **I** liegt im Bereich von 1 zu 1 bis 1 zu 100, insbesondere 1 zu 1 bis 1 zu 20, ganz besonders im Bereich von 1 zu 2 bis 1 zu 5.

Das molare Verhältnis von Bisphosphit **I** zu sterisch gehindertem sekundären Amin **II** liegt im Bereich von 0,1 zu 10 bis 10 zu 1, insbesondere im Bereich von 5 zu 10 bis 10 zu 5, ganz besonders im Bereich von 0,8 zu 1 bis 1 zu 0,8.

Die Konzentration des Rhodiums im Reaktionsgemisch liegt im Bereich von 1 bis 1000 Massen-ppm, insbesondere im Bereich von 20 bis 300 Massen-ppm, ganz besonders im Bereich von 40 bis 150 Massen-ppm.

Der Rhodiumkatalysator kann als aktiver Komplex in den Prozess eingebracht werden. Bevorzugt wird der aktive Komplex im Hydroformylierungsreaktor unter Hydroformylierungsbedingungen in Gegenwart des Bisphosphit **I** aus stabilen, leicht lagerbaren Rhodiumverbindungen hergestellt. Geeignete Rhodiumverbindungen dafür sind zum Beispiel Rhodium(II)- und Rhodium(III)-salze, wie Rhodium(III)chlorid, Rhodium(III)nitrat, Rhodium(III)sulfat, Kalium-Rhodiumsulfat, Rhodium(II)- bzw. Rhodium(III)-carboxylat, Rhodium(II)- und Rhodium(III)acetat, Rhodium(II)octanoat, Rhodium(II)nonanoat, Rhodium(III)oxid, Salze der Rhodium(III)säure, Trisammoniumhexachtororhodat(III). Weiterhin eignen sich Rhodiumkomplexe, wie Rhodiumbiscarbonylacetylacetonat, Acetylacetonatobisethylenrhodium(I). Besonders geeignet sind Rhodiumacetat, Rhodiumoctanoat und Rhodiumnonanoat.

Das Katalysatorsystem, bestehend aus mindestens einen Rhodiumkomplex, Ligand, Amin und anderen Rhodiumverbindungen ist vorzugsweise im Hydroformylierungsgemisch, bestehend aus Einsatzkohlenwasserstoffgemisch und Produkten (C₅-Aldehyde, C₅-Alkohole, Hochsieder) gelöst. Optional kann zusätzlich ein Lösungsmittel, z. B. Toluol, Esterderivate des 2,2,4-Trimethylpentan-1,3-diols (Texanol^{®}), eutektisches Gemisch aus Biphenyl und Diphenylether (Diphyl^{®}), hochsiedende Rückstände, Phthalate, wie Di(2-ethylhexyl)phthalat oder Dinonylphthalat, oder Ester von 1,2-Cyclohexansäuren oder Ester der Benzoesäure, wie zum Beispiel Isononylbenzoat, verwendet werden.

### Verfahrensdurchführung

Im erfindungsgemäßen Verfahren wird die Hydroformylierung bei Temperaturen im Bereich von 70 bis 150°C, vorzugsweise von 80 bis 130 °C, insbesondere im Bereich von 100 bis 120°C durchgeführt.

Der Reaktionsdruck beträgt 1 bis 20 MPa, vorzugsweise 1,0 bis 3,5 MPa und insbesondere 1,5 bis 2,1 MPa.

Die Hydroformylierung wird bevorzugt kontinuierlich durchgeführt. Als Reaktionsgefäße können beispielsweise Blasensäulenreaktoren oder Rührkessel verwendet werden.

Die Hydroformylierungsprodukte können durch destillative Aufarbeitung des flüssigen Hydroformylierungsgemisch abgetrennt werden. Die aus dem Reaktor abgezogene gasförmige Phase wird zum Teil kondensiert. Der weiterhin gasförmige Anteil wird zum größten Teil in das Reaktionssystem zurückgeführt. Anschließend erfolgt eine destillative Trennung der flüssigen Produkte.

Vorzugsweise werden im erfindungsgemäßen Verfahren die Reaktionsprodukte, insbesondere die C₅-Aldehyde, mit Reaktionsgas aus dem Reaktor ausgetragen. Das Reaktionsgas besteht im Wesentlichen aus nicht umgesetzten Synthesegas, nicht umgesetzten Edukten und C₅ Aldehyden, wobei das Edukt auch einen inerten Anteil an gesättigten Kohlenwasserstoffen enthalten kann. Dazu werden je t Einsatzstoff 500 bis 9000 Nm³ Reaktionsgas mit Hilfe von mindestens einer Düse in die flüssige Phase im Reaktor eingeleitet. Die relative Menge des Reaktionsgases ist abhängig vom Flüssigkeitsstand im Reaktor, der 20 bis 80 % der Reaktorhöhe betragen soll. Das heißt, dass bei hohem Flüssigkeitsstand die Reaktionsgasmenge erhöht und bei niedrigem Stand verringert wird.

Das den Reaktor verlassende Reaktionsgas, das mit Edukt und Produkten beladen ist, wird über einen Aerosolbrecher (Demister) geleitet, in dem die im Gasstrom enthaltende Flüssigkeitströpfchen abgeschieden werden. Die dabei anfallende Flüssigkeit wird in den Reaktor zurückgeleitet. Durch diese Vorrichtung kann der Austrag von Katalysator mit der Gasphase minimiert werden. Aus dem Gasgemisch werden Produkte und Edukte auskondensiert und das Synthesegas, gegebenenfalls nach Ausschleusung einer Teilmenge, in den Reaktor zurückgefahren. Das Kondensat wird in einer oder mehreren Kolonnen in Produkte und Edukte getrennt.

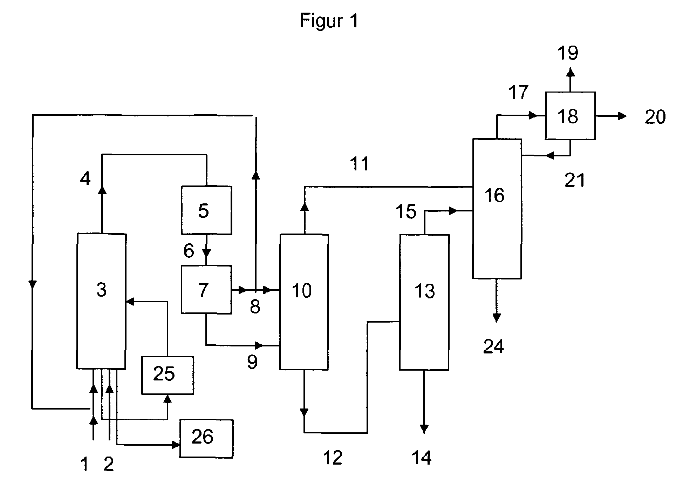

Wird als Edukt ein Kohlenwasserstoffgemisch verwendet, das praktisch nur aus n-Butan und linearen Butenen, insbesondere 2-Butenen, besteht, erfolgt die Durchführung bevorzugt auf folgende Weise, die anhand eines Blockschemas einer Anlage, in der das erfindungsgemäße Verfahren durchgeführt werden kann, erläutert wird (**Figur 1**).

Synthesegas (1), ein Teil der abgetrennten Gasphase (8) und Edukt (2) werden in den Hydroformylierungsreaktor (3) eingeleitet, in dem eine flüssige Phase vorliegt. Ein gasförmiger Strom (4), bestehend aus Synthesegas, Edukt und Produkte, wird abgezogen, in der Vorrichtung (5) teilkondensiert und im Trennbehälter (7) in eine flüssige Phase (9) und eine Gasphase (8) getrennt. Ein Teil der Gasphase(8) wird nach Kompression in den Reaktor (3) zurückgeführt. Der andere Teil von Strom (8) und die flüssige Phase (9) werden in die Destillationskolonne (10) eingeleitet, in der ein Kopfprodukt, bestehend aus C₄-Kohlenwasserstoffen und Synthesegas, abgetrennt wird. Das Sumpfprodukt (12) wird in der Kolonne (13) in ein Sumpfprodukt (14), das die gebildeten C₅-Aldehyde und andere Produkte enthält, und ein Kopfprodukt (15) getrennt. Die beiden Kopfprodukte (11) und (15) werden in der Kolonne (16) aufgetrennt. Dabei fällt als Sumpfprodukt ein C₄-Kohlenwasserstoffgemisch an. Das Kopfprodukt (17) wird teilkondensiert und im Trenngefäß (18) in eine Gasphase (19), hauptsächlich Synthesegas, eine Wasserphase (20) und eine flüssige C₄-Kohlenwasserstoffphase (21), die in die Kolonne (16) zurückgefahren wird, getrennt.
Die Gasphase (19) kann optional teilweise in den Reaktor (3) zurückgefahren werden. Ebenfalls kann es zweckmäßig sein einen Teil des Kohlenwasserstoffstroms (24) in den Reaktor (3) zurückzuführen. Gegebenfalls können die Vorrichtungen (5) und (7) aus einem Apparat bestehen. Eine weitere Option ist, die Kolonnen (10) und (13) durch eine Kolonne zu ersetzen. Optional wird ein Teil des Reaktorinhaltes über ein Filter (25) unter Reaktionsdruck gekreist, um unlösliche Folgeprodukte des Bisphosphit-Liganden **I** aus dem Reaktionssystem zu entfernen.
Um eine Synthesegasverarmung in der Reaktionslösung zu verhindern, muss die Verweilzeit in der Filtrationseinheit begrenzt werden. Die Verweilzeit in der Filtrationseinheit liegt im Bereich von 5-10 Minuten, insbesondere im Bereich von 2-5 Minuten, ganz besonders unter 2 Minuten.
Als weitere Option wird der Zulauf zu dieser Filtrationseinheit (25) abgekühlt. Dabei wird eine Temperaturabsenkung des Zulaufs von 5-20 °C, insbesondere eine Temperaturabsenkung von 20 - 30 °C, ganz besonders eine Temperaturabsenkung von 30-40 °C unterhalb der Reaktionstemperatur, angestrebt.
Die Filtration kann kontinuierlich oder diskontinuierlich erfolgen.

Im Folgenden werden die einzelnen Aufarbeitungsschritte des Hydroformylierungsgemisches genauer beschrieben.

### Abtrennung mitgerissener Flüssigkeitströpfchen

Der gasförmige Reaktionsaustrag enthält feinste Flüssigkeitströpfchen, die das Katalysatorsystem enthalten. Zur Minimierung der Katalysatorverluste, insbesondere des Rhodiums, werden die Tröpfchen mittels eines Aerosolbrechers von der Gasphase abgetrennt. Als Aerosolbrecher werden Behälter mit Einbauten verwendet z. B. Demister, Zyklonabscheider, Lamellenabscheider, Füllkörper, Filterkerzen.

Der Aerosolbrecher wird vorzugsweise bei Reaktordruck und Reaktortemperatur betrieben. Er ist so groß ausgelegt, dass über 99,9 %, insbesondere über 99,99 % der mitgerissenen Flüssigkeit abgeschieden wird. Die abgetrennte Flüssigkeit wird in den Reaktor zurückgeleitet. In der den Aerosolbrecher verlassenden Gasphase liegt der Rhodiumgehalt unter der Nachweisgrenze von 0,1 ppm

### Kondensation und Phasentrennung

Die Partialkondensation des gasförmigen Reaktionsgemisches kann in üblichen technischen Kondensatoren, beispielsweise Rohrbündel- oder Plattenwärmeaustauscher erfolgen. Die Kondensation wird im Temperaturbereich von 3 bis 90 °C, insbesondere im Bereich von 15 bis 60 °C durchgeführt. Als Kühlmittel zur Abführung der Kondensationswärme können beispielsweise Luft, Wasser oder Kühlsole eingesetzt werden. Der Druck ist dabei geringer als im Hydroformylierungsreaktor und niedriger als der Druck in der nachfolgenden Kolonne.

Nach der Partialkondensation wird in einem nachgeschalteten Behälter die flüssige Phase von der Gasphase getrennt.

Die Gasphase enthält den größten Teil des überschüssigen Synthesegases. Daneben enthält sie gemäß der Partialdrücke der Komponenten in der flüssigen Phase weitere Stoffe. In der Gasphase sind neben Synthesegas hauptsächlich C₄-Kohlenwasserstoffe und im geringeren Ausmaß C₅-Aldehyde enthalten.

Die Gasphase wird auf die Saugseite eines Gasverdichters geleitet. Der größere Anteil (ca. 99 %) der Gasphase wird in den Hydroformylierungsreaktor zurückgefahren. Der andere Teil wird nach dem Verdichter in eine Trennkolonne (10) eingespeist. Es ist zweckmäßig zum Schutze des Verdichters, im Gas enthaltende Flüssigkeitströpfchen abzustreifen, beispielsweise unter Verwendung eines Aerosolbrechers.

Die flüssige Phase enthält den größten Teil der Produkte, Edukte und gelöstes Synthesegas.
Beispiel für die Zusammensetzung der flüssigen Phase:
Butan 50 bis 60 %, Buten 3 bis 10 %, 30 bis 40 % Pentanal, 2 bis 8 % 2-Methylbutanal, 0,5 bis 2 % CO, < 1 % Wasser. Sie wird in Kolonne (10) eingeleitet.

### Destillative Aufarbeitung in den Kolonnen (10), (13),(16)

Die Auftrennung des Stoffgemisches erfolgt vorzugsweise in drei Kolonnen. Diese Kolonnen können mit Einbauten, die z. B. aus Böden, rotierenden Einbauten, regellosen Schüttungen und/oder geordneten Packungen sind, versehen sein.

Bei den Kolonnenböden können z. B. folgende Typen zum Einsatz kommen:
- Böden mit Bohrungen oder Schlitzen in der Bodenplatte.
- Böden mit Hälsen oder Kaminen, die von Glocken, Kappen oder Hauben überdeckt sind.
- Böden mit Bohrungen in der Bodenplatte, die von beweglichen Ventilen überdeckt sind.
- Böden mit Sonderkonstruktionen.

In Kolonnen mit rotierenden Einbauten kann der Rücklauf z. B. durch rotierende Trichter versprüht oder mit Hilfe eines Rotors als Film auf einer beheizten Rohrwand ausgebreitet werden.

In dem erfindungsgemäßen Verfahren können, wie bereits gesagt, Kolonnen eingesetzt werden, die regellose Schüttungen mit verschiedenen Füllkörpern aufweisen. Die Füllkörper können aus fast allen Werkstoffen, insbesondere aus Stahl, Edelstahl, Kupfer, Kohlenstoff, Steingut, Porzellan, Glas oder Kunststoffen bestehen und die verschiedensten Formen, insbesondere die Form von Kugeln, Ringen mit glatten oder profilierten Oberflächen, Ringen mit Innenstegen oder Wanddurchbrüchen, Drahtnetzringen, Sattelkörper und Spiralen aufweisen.

Packungen mit regelmäßiger/geordneter Geometrie können z. B. aus Blechen oder Geweben bestehen. Beispiele für solche Packungen sind Sulzer Gewebepackungen BX aus Metall oder Kunststoff, Sulzer Lamellenpackungen Mellapak aus Metallblech, Hochleistungspackungen von Sulzer wie Mella-pakPlus, Strukturpackungen von Sulzer (Optiflow), Montz (BSH) und Kühni (Rombopak).

### Vortrennung in Kolonne (10)

In der Kolonne (10) wird der Teil der Gasphase (8), der nicht in den Reaktor zurückgeleitet wird und die flüssige Phase (9) in ein Sumpfprodukt (12), bestehend aus Produkten und einem geringen Anteil an C₄-Kohlenwasserstoffen, und ein Kopfprodukt (11), das Synthesegas und den größten Teil der C₄-Kohlenwasserstoffe, jedoch keine Produkte enthält, getrennt.

Die Kolonne (10) weist 10 bis 30 theoretische Trennstufen, insbesondere 25 bis 35 theoretische Trennstufen auf. Die flüssige Phase wird in die 15. bis 25. theoretische Trennstufe, insbesondere in die 20. bis 22. theoretische Trennstufe eingeleitet. Die Gasphase (8) wird in die 15. bis 25. theoretische Trennstufe, insbesondere in die 20. bis 22. theoretische Trennstufe eingespeist. (Bodennummerierung oben 1 → unten 25)

Die Zuführungsströme haben Temperaturen im Bereich von 10 bis 125 °C, insbesondere im Bereich von 40 bis 100 °C, insbesondere im Bereich von 80 bis 90 °C.

Kolonne (10) wird vorzugsweise bei einem Druck zwischen 1 und 2 MPa, insbesondere bei einem Druck im Bereich von 1,6 bis 1,8 MPa betrieben.

Die Kopftemperatur und Sumpftemperatur ist druckabhängig. Bei einem Druck von 1,6 bis 1,8 MPa liegt die Kopftemperatur zwischen 95 und 105°C und die Sumpftemperatur zwischen 125 und 140 °C.

Das Rücklaufverhältnis bezogen auf die unter diesen Bedingungen kondensierbaren Anteile (C₄-Kohlenwasserstoffe) liegt im Bereich von 0,5 bis 3, insbesondere im Bereich von 1 bis 2.

### Trennung in Kolonne (13)

In Kolonne (13) werden aus dem Sumpfprodukt (12) die restlichen C₄-Kohlenwasserstoffe als Kopfprodukt abgetrennt.

Die Kolonne (13) weist 5 bis 20 theoretische Trennstufen, insbesondere 8 bis 15 theoretische Trennstufen auf. Die flüssige Phase (12) wird in die 5. bis 12. theoretische Trennstufe, insbesondere in die 6. bis 9. theoretische Trennstufe eingeleitet.

Kolonne (13) wird vorzugsweise bei einem Druck zwischen 0,1 und 0,8 MPa, insbesondere bei einem Druck im Bereich von 0,4 MPa betrieben.

Die Kopftemperatur und Sumpftemperatur sind druckabhängig. Bei einem Druck von 0,4 MPa liegt die Kopftemperatur bei 45°C und die Sumpftemperatur bei 160 °C.

Das Rücklaufverhältnis liegt im Bereich von 0,5 bis 3, insbesondere im Bereich von 1 bis 2.

Als Sumpfprodukt (14) wird ein Gemisch erhalten, das zu über 95 %, insbesondere über 97 %, ganz besonders über 98 % aus C₅-Aldehyden besteht. Weiterhin kann dieses Gemisch Pentanole und Hochsieder, gebildet aus den Pentanalen, enthalten. Der Gehalt an n-Pentanal ist mindestens 90 %.

### Trennung in Kolonne (16)

Kolonne (16) dient zur Trennung von Synthesegas, C₄-Kohlenwasserstoffen und Wasser, das durch die Edukte eingeschleppt und/oder durch Nebenreaktionen, beispielsweise Aldolkondensation von Aldehyden, entstanden ist.

Die Kolonne (16) weist 5 bis 25 theoretische Trennstufen, insbesondere 10 bis 20 theoretische Trennstufen auf. Die Kopfprodukte (11) und (15) der beiden Kolonnen (10) und (13) werden in die 1. bis 5. theoretische Trennstufe, insbesondere in die 1. bis 3. theoretische Trennstufe eingeleitet. Die Zulauftemperatur liegt vorzugsweise zwischen 40 und 100 °C.

Die Kolonne (16) wird vorzugsweise bei 1,5 MPa betrieben. Die Sumpftemperatur liegt dabei bei 100 °C. Die Kopftemperatur bei ca. 93 °C

Das Kopfprodukt (17) wird kondensiert und nach Abkühlen auf etwa 5 °C im Trennbehälter (18) in eine Gasphase (19), eine Wasserphase (20) und eine organische Phase (21) getrennt, die als Rücklauf in Kolonne (16) eingeleitet wird.

Alternativ dazu kann Strom (17) mit den Kopfprodukten (11) und (15) vereinigt werden. Nach Abkühlen auf 5 °C wird dieser Strom in eine Gasphase (19a), eine Wasserphase (20a) und eine organische Phase getrennt, die in Kolonne (16) als Rückfluss auf den Kopf eingeleitet wird.

### Verwendung der Ströme (19), (20) und (24)

Die Gasphase (19) besteht aus Synthesegas, Inertgas, wie beispielsweise Stickstoff oder Kohlendioxid, und Wasserdampf. Sie kann abhängig vom Inertgasanteil teilweise in den Hydroformylierungsreaktor zurückgeführt (3) werden. Der andere Teil, bevorzugt die gesamte Menge kann thermisch oder stofflich verwertet werden. Das abgetrennte Gas kann beispielsweise als Heizgas verwendet werden. Eine andere Nutzung des Gases ist beispielsweise die Gewinnung von Wasserstoff, beispielsweise durch Konvertierung.

Das abgetrennte Wasser (20) wird verworfen oder gegebenenfalls als Prozesswasser verwendet.

Das Kohlenwasserstoffgemisch (24) besteht hauptsächlich aus Butan. Der Butangehalt ist abhängig vom Butangehalt im Edukt und Umsatz der linearen Butene. Bevorzugt ist der Butangehalt größer als 80 %, insbesondere größer als 90 %. Darüber hinaus kann Strom (24) geringe Mengen an Produkten, insbesondere n-Pentanal, enthalten. Der Gehalt an Produkten ist kleiner 10 Massen-ppm, insbesondere kleiner als 1 Massen-ppm.
Ein Teil des Stroms (24) kann in den Hydroformylierunysreaktor (3) zurückgeführt werden mit der Maßgabe, dass das in den Hydroformylierungsreaktor (3) eingeleitete Gesamt-Kohlenwasserstoffgemisch (Summe aus Frisch-Edukt (2), Kohlenwasserstoffe aus den anteilig zurückgeführten Strömen (8) und (24)) einen Gehalt an linearen Butenen von größer als 15 Massen-%, insbesondere von größer als 20, ganz besonders größer als 25 aufweist. Dieser Strom ist nicht in Figur 1 dargestellt.

Strom (24) kann zu Heizzwecken verwendet werde. Ohne weitere Aufarbeitung kann es zur Herstellung von Acetylen oder Synthesegas verwendet werden. Nach Hydrierung der Butene kann es zu reinem Butan aufgearbeitet werden, das als Treibmittel für Aerosole Verwendung findet. Weiterhin wird reines n-Butan zur Herstellung von Maleinsäureanhydrid eingesetzt.

### Verwendung des Zielproduktes (14)

Das Sumpfprodukt (14), das wie bereits beschrieben, aus C₅-Aldehyden, geringen Mengen an C₅-Alkoholen und weniger als 1 % an Hochsiedern besteht, kann als solches verwendet, weiter verarbeitet oder destillativ aufgetrennt werden. Durch Destillation kann reines n-Pentanal (Sp. 102-103°C), reines 2-Methylbutanal (Sp. 91 - 92 °C), 2-Methylbutanol (Sp. 129°C) und n-Pentanol (Sp. 138°C) gewonnen werden.

Die C₅-Aldehyde können zu den entsprechenden Alkoholen hydriert werden. Ihre Oxidation liefert die entsprechenden Carbonsäuren.

Durch Aldolkondensation der C₅-Aldehyde kann ein Decenalgemisch hergestellt werden. Hierzu kann als Edukt zusätzlich ein C₅-Aldehydstrom, der aus einem alternativen Herstellungsverfahren stammt, wie z. B. der parallel eingereichten Anmeldung "Verfahren zur Abtrennung von 1-Buten aus C₄-haltigen Kohlenwasserstoffströmen durch Hydroformylierung", verwendet werden. Die Aldolkondensation kann nach an sich bekannten Verfahren durchgeführt werden. Insbesondere erfolgt sie, wie in DE 199 57 522 beschrieben:
Das Decenal- wird zu einem Decanolgemisch, einem begehrten Weichmacheralkohol, hydriert. Die Hydrierung erfolgt ebenfalls nach an sich bekannten Verfahren, beispielsweise im Temperaturbereich von 170 bis 200°C bei einem Druck von 15 bis 30 bar an einem Trägerkatalysator, der als aktive Komponenten, Nickel, Kupfer und Chrom enthält.

Vorzugsweise wird ein Decanolgemisch mit einem 2-Propylheptanolgehalt von mindestens 90 % hergestellt. Bei einem n-Pentanal/2-Methylbutanal-Verhältnis von kleiner 90 zu 10 wird dann vor der Aldolkondensation ein Teil des 2-Methylbutanal abgetrennt.
Weiterhin kann aus dem Sumpfprodukt (14) durch Aldolkondensation, Selektivhydrierung des Kondensats zu Decanal und anschließender Oxidation ein Decansäuregemisch mit einem hohen Anteil an 2-Propylheptansäure erzeugt werden.

### Aufarbeitung der flüssigen Reaktorphase

Nach Beendung der Produktionsphase kann der Gehalt an Butan, Butenen und Aldehyden im Reaktionsgemisch mit Hilfe des Kreisgases reduziert werden. Anschließend kann der Reaktorinhalt über einen Dünnschichtverdampfer eingeengt werden. Das Verhältnis von Destillat und Sumpf sollte 2:1, bevorzugt 5 : 1 und insbesondere von 7 : 1 oder höherer betragen. Der Sumpfstrom muss noch bei Raumtemperatur fließfähig sein und darf keinerlei Niederschläge aufweisen. Mindestens 90 % des im Einsatzstrom befindlichen Rhodiums ist in dem Sumpfprodukt vorhanden. Der so aufbereitete Rhodiumhaltige Sumpf kann einer Rhodium-Rückgewinnung zugeführt werden. Die Destillatphase kann der weiteren stofflichen Verwertung zugeführt werden und z. B. eine Verwendung als hochsiedendes Lösemittel, als Einsatzstoff zur Synthesegaserzeugung, als Einsatzbrennstoff zur Schwefelsäureherstellung oder im Kraftstoffsektor finden. Optional kann die flüssige Reaktorphase im laufenden Betrieb aus dem Reaktor ausgeschleust werden und der oben beschriebenen Aufarbeitung zugeführt werden.

Die folgenden Beispiele sollen die Erfindung erläutern, ohne die Anwendungsbreite einzuschränken, die sich aus der Beschreibung und den Patentansprüchen ergibt.

### Beispiele

Eine Hydroformylierung von Buten/Butan-Mischungen wurde in einer kontinuierlich betriebenen Versuchsanlage durchgeführt.
Diese Versuchsanlage bestand im Wesentlichen aus einem 20 Liter fassenden Druckreaktor mit einem nachgeschalteten Kondensator und Phasentrennbehälter (Gas/Flüssigkeit) für die aus dem Reaktor stammende Gasphase sowie einem Kreisgasverdichter, der die Gasphase aus dem Phasentrennbehälter wieder unten in die Reaktionszone zurück führt. Ein Teil dieses Kreisgases wird nach der Phasentrennung als Abgas aus dem Reaktionssystem gefahren.
Um eine optimale Gasverteilung im Reaktorsystem zu realisieren, wurde hier ein Gasverteilerring mit Bohrungen verbaut.
Über installierte Heiz- und Kühlvorrichtungen konnte der Reaktor temperiert werden.

Vor der Hydroformylierung wurde das Reaktorssystem mit Stickstoff frei von Sauerstoff gespült. Anschließend wurde der Reaktor mit 12 Liter Katalysatorlösung gefüllt.

Diese Katalysatorlösung setzte sich aus 12 kg eines eutektischen Gemisches aus Biphenyl und Diphenylether (Diphyl^{®} Wärmeträgeröl der Fa. Lanxess), 3 g Rh(acac)(CO)₂, 36 g Bisphosphit-Ligand **I,** 67,5 g Amin **IIb** zusammen und wurde vorher in einem Behälter gemischt. Das eutektische Gemisch aus Biphenyl und Diphenylether (Diphyl^{®}) wurde zuvor mit Stickstoff gestrippt, um Sauerstoff und Wasser aus dem Wärmeträgeröl zu entfernen.
Anschließend wurde das Reaktorsystem mit Synthesegas frei von Stickstoff gespült. Nachdem der Stickstoffgehalt < 10 Vol% gefallen war, wurde das Reaktorsystem mit Synthesegas auf 1,0 MPa aufgedrückt und anschließend auf 120 °C aufgeheizt. Nach Erreichen der Betriebstemperatur wurde das Reaktorsystem mit Synthesegas auf 1,7 MPa Reaktionsdruck gebracht.
Danach wurde die Zugabe der Ausgangsstoffe gestartet. Das Rohbutan wurde über einen Verdampfer gefahren, um das Rohbutan gasförmig in das Kreisgas zu fahren.

Folgende Durchsätze wurden eingestellt:
0,3 kg/h Rohbutan (eine Mischung aus 35 % 2-Butenen und n-Butan und 1-Buten Konzentrationen von ca. 1 %) 75 NI/h Synthesegas (50 Vol% H₂ und 50 Vol% CO) Zur täglichen Dosierung des Bisphosphit-Liganden **I** und Amins **IIb** wurde eine 1,4%ige Lösung des Bisphosphit-Liganden **I** in n-Pentanal angesetzt, welches zuvor durch Strippen mit Stickstoff von restlichen C₄-Kohlenwasserstoffen (< 3 %) befreit wurde. Das Amin **IIb** wurde in einem dreifachen molaren Überschuss zum Bisphosphit-Liganden **I** eingesetzt. Zur besseren Stabilisierung dieser Lösung wurde das Amin **IIb** vor dem Bisphosphit-Liganden **I** zur Lösung gegeben.

Nach ca. 1000 h wurde ein stationärer Zustand erreicht. Die Reaktionsprodukte wurden kontinuierlich über den Kreisgasstrom aus dem Reaktor entfernt und im Kondensator bei 50 °C partiell auskondensiert. Die auskondensierte Phase wurde kontinuierlich aus dem Phasentrennbehälter gefahren. Zur Umsatzbestimmung wurden aus dem Kreisgas vor und nach Reaktor Proben gezogen.
Durch eine tägliche Dosierung von 100 g der oben beschriebenen Lösung, konnte der Umsatz und die Regioselektivität konstant gehalten werden.

Zur Bestimmung des Reaktorinhaltes wurden Proben aus dem Reaktor entnommen und mittels Flüssigchromatographie (HLPC) untersucht.

Unter den gewählten Reaktionsbedingungen wurden Butenumsätze von rund 65 - 70 % erzielt. Die prozentuale Verteilung zwischen n-Pentanal und 2-Methylbutanal, bzw. die n/iso-Selektivität, betrug 95 % zu 5 %.
In der stationären Phase des Versuches konnte kein Rhodiumabbau verzeichnet werden.

Nach Ende des Versuches wurde der Reaktor entspannt und die Katalysatorlösung untersucht. Im Reaktor zeigte sich ein Niederschlag.
Eine Analyse dieses Niederschlages ergab, dass diese aus phosphorhaltigen Folgeprodukten des Bisphosphit-Liganden **I** und dem eingesetzten Amin **IIb** bestanden.
Es wurden keinerlei Anbackungen dieser Ausfällungen in dem Reaktor festgestellt. Ein Teil des Reaktorinhaltes wurde, nach Abtrennung des Niederschlages, bei 1,2 KPa abs. und 220 °C Sumpftemperatur auf 13 % bezogen auf die Einsatzmasse eingeengt. Der erhaltene Rückstand aus dem Sumpf war noch fließfähig und es wurde kein Niederschlag festgestellt.
Eine Rhodiumanalyse zeigte, dass das sich das gesamte Rhodium aus der Einsatzmasse in diesem Sumpfrückstand befand.

## Patentansprüche

1. Verfahren zur Herstellung von C₅-Aldehydgemischen aus einem lineare Butene enthaltenden Kohlenwasserstoffgemisch durch endständige Hydroformylierung unter isomerisierenden Bedingungen, wobei ein Katalysatorsystem eingesetzt wird, enthaltend Rhodium, ein Bisphosphit-Ligand der Formel **I** 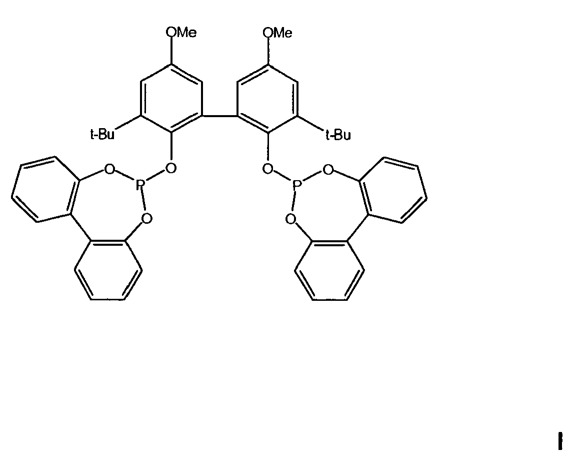 und ein Amin der Formel **II** 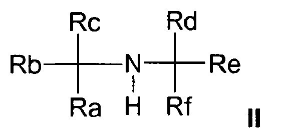 wobei Ra, Rb, Rc, Rd, Re und Rf gleiche oder unterschiedliche Kohlenwasserstoffreste, die auch untereinander verbunden sein können, sind.

2. Verfahren nach Anspruch 1 **dadurch gekennzeichnet, dass** das Kohlenwasserstoffgemisch bis zu 5 Massen-% bezogen auf die C₄-Olefinfraktion an Isobuten enthält.

3. Verfahren nach den Ansprüchen 1 und 2 **dadurch gekennzeichnet, dass** das Kohlenwasserstoffgemisch Restgehalte an 1-Buten im Verhältnis zu den isomeren 2-Butenen von weniger als 1 zu 10 enthält.

4. Verfahren nach den Ansprüchen 1 bis 3 **dadurch gekennzeichnet, dass** das molare Verhältnis von Rhodium zu dem Bisphosphit der Formel **I** in dem Bereich von 1 zu 2 bis 1 zu 5 liegt.

5. Verfahren nach den Ansprüchen 1 bis 4 **dadurch gekennzeichnet, dass** das molare Verhältnis von Bisphosphit der Formel **I** zu Amin der Formel **II** in dem Bereich 0,1 zu 10 bis 10 zu 1 liegt.

6. Verfahren nach den Ansprüchen 1 bis 5 **dadurch gekennzeichnet, dass** die Konzentration des Rhodiums im Reaktionsgemisch von 1 Massen-ppm bis zu 1000 Massen-ppm bezogen auf das Gesamtgewicht des Reaktionsgemischs beträgt.

7. Verfahren nach den Ansprüchen 1 bis 6, **dadurch gekennzeichnet, dass** das Kohlenwasserstoffgemisch, enthaltend lineare Butene, mit einer n-/iso-Selektivität von mindestens 90 % hydroformyliert wird.

8. Verfahren nach den Ansprüchen 1 bis 7 **dadurch gekennzeichnet, dass** die Reaktion in einem Temperaturbereich von 70 bis 150°C durchgeführt wird.

9. Verfahren nach den Ansprüchen 1 bis 8 **dadurch gekennzeichnet, dass** die Reaktion in einem Druckbereich von 1 bis 20 MPa durchgeführt wird.

10. Verfahren nach den Ansprüchen 1 bis 9 **dadurch gekennzeichnet, dass** die Reaktion kontinuierlich durchgeführt wird.

11. Verfahren nach den Ansprüchen 1 bis 10, **dadurch gekennzeichnet, dass** ausgefallene Reaktionsprodukte durch Filtration unter Reaktionsdruck aus einem Reaktorkreislauf abgetrennt werden.

12. Verfahren nach Anspruch 11, **dadurch gekennzeichnet, dass** die Verweilzeit der Reaktionslösung während der Filtration kleiner als 5 Minuten ist.

13. Verfahren nach den Ansprüchen 11 und 12, **dadurch gekennzeichnet, dass** die Temperatur des Zulaufs zur Filtration in einem Bereich zwischen 5 bis 40 °C unterhalb der Reaktionstemperatur abgesenkt wird.

14. Verfahren nach den Ansprüchen 11 bis 13, **dadurch gekennzeichnet, dass** die Filtration kontinuierlich durchgeführt wird.

15. Verfahren nach den Ansprüchen 1 bis 14, **dadurch gekennzeichnet, dass** das Reaktionsgemisch in C₅-Aldehyde, Synthesegas, Kohlenwasserstoffe und einen Rhodium-haltigen Rückstand aufgetrennt wird.

## Claims

1. Process for preparing C₅ aldehyde mixtures from a hydrocarbon mixture comprising linear butenes by terminal hydroformylation under isomerizing conditions, using a catalyst system comprising rhodium, a bisphosphite ligand of the formula **I** 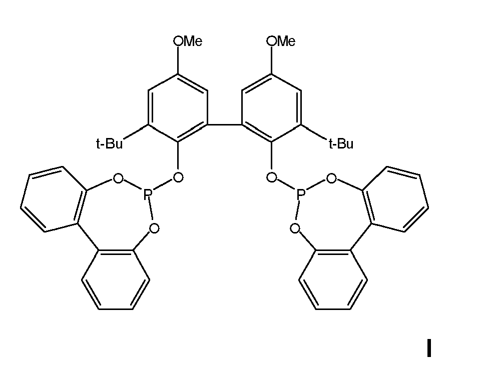 and an amine of the formula **II** 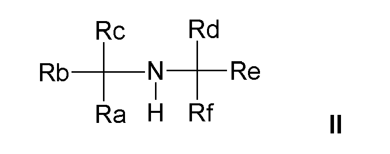 where Ra, Rb, Rc, Rd, Re and Rf are identical or different hydrocarbon radicals which may also be joined to one another.

2. Process according to Claim 1, **characterized in that** the hydrocarbon mixture contains up to 5% by mass, based on the C₄ olefin fraction, of isobutene.

3. Process according to either of Claims 1 and 2, **characterized in that** the hydrocarbon mixture comprises residual contents of 1-butene in relation to the isomeric 2-butenes of less than 1:10.

4. Process according to any one of Claims 1 to 3, **characterized in that** the molar ratio of rhodium to the bisphosphite of the formula I is in the range from 1:2 to 1:5.

5. Process according to any one of Claims 1 to 4, **characterized in that** the molar ratio of bisphosphite of the formula I to amine of the formula II is in the range of 0.1:10 to 10:1.

6. Process according to any one of Claims 1 to 5, **characterized in that** the concentration of the rhodium in the reaction mixture is 1 ppm by mass up to 1000 ppm by mass based on the total weight of the reaction mixture.

7. Process according to any one of Claims 1 to 6, **characterized in that** the hydrocarbon mixture comprising linear butenes is hydroformylated with an n/iso selectivity of at least 90%.

8. Process according to any one of Claims 1 to 7, **characterized in that** the reaction is carried out within a temperature range from 70 to 150°C.

9. Process according to any one of Claims 1 to 8, **characterized in that** the reaction is carried out within a pressure range from 1 to 20 MPa.

10. Process according to any one of Claims 1 to 9, **characterized in that** the reaction is carried out continuously.

11. Process according to any one of Claims 1 to 10, **characterized in that** precipitated reaction products are removed from a reactor circulation system by filtration under reaction pressure.

12. Process according to Claim 11, **characterized in that** the residence time of the reaction solution during the filtration is less than 5 minutes.

13. Process according to either of Claims 11 and 12, **characterized in that** the temperature of the feed to the filtration is within a range between 5 and 40°C below the reaction temperature.

14. Process according to any one of Claims 11 to 13, **characterized in that** the filtration is carried out continuously.

15. Process according to any one of Claims 1 to 14, **characterized in that** the reaction mixture is separated into C₅ aldehydes, synthesis gas, hydrocarbons and a rhodium-containing residue.

## Revendications

1. Procédé pour la préparation de mélanges de C₅-aldéhydes à partir d'un mélange d'hydrocarbures contenant des butènes linéaires par hydroformylation en position d'extrémité dans des conditions d'isomérisation, un système de catalyseur étant utilisé, contenant du rhodium, un ligand biphosphite de formule I 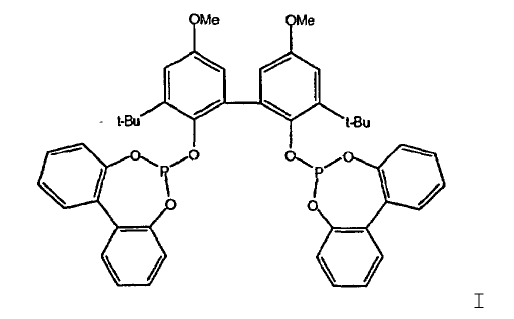 et une amine de formule II 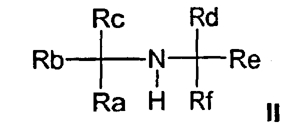 où Ra, Rb, Rc, Rd, Re et Rf représentent des radicaux hydrocarbonés identiques ou différents, qui peuvent également être liés entre eux.

2. Procédé selon la revendication 1, **caractérisé en ce que** le mélange d'hydrocarbures contient jusqu'à 5% en masse, par rapport à la fraction d'oléfines en C₄, d'isobutène.

3. Procédé selon les revendications 1 et 2, **caractérisé en ce que** le mélange d'hydrocarbures contient des teneurs résiduelles en 1-butène, par rapport aux 2-butènes isomères, inférieures à 1:10.

4. Procédé selon les revendications 1 à 3, **caractérisé en ce que** le rapport molaire de rhodium à biphosphite de formule I se situe dans la plage de 1:2 à 1:5.

5. Procédé selon les revendications 1 à 4, **caractérisé en ce que** le rapport molaire de biphosphite de formule I à amine de formule II se situe dans la plage de 0,1:10 à 10:1.

6. Procédé selon les revendications 1 à 5, **caractérisé en ce que** la concentration en rhodium dans le mélange réactionnel est de 1 ppm en masse à 1000 ppm en masse par rapport au poids total du mélange réactionnel.

7. Procédé selon les revendications 1 à 6, **caractérisé en ce que** le mélange d'hydrocarbures, contenant des butènes linéaires, est hydroformylé à une sélectivité n/iso d'au moins 90%.

8. Procédé selon les revendications 1 à 7, **caractérisé en ce que** la réaction est réalisée dans une plage de température de 70 à 150°C.

9. Procédé selon les revendications 1 à 8, **caractérisé en ce que** la réaction est réalisée dans une plage de pression de 1 à 20 MPa.

10. Procédé selon les revendications 1 à 9, **caractérisé en ce que** la réaction est réalisée en continu.

11. Procédé selon les revendications 1 à 10, **caractérisé en ce que** les produits de réaction précipités sont séparés par filtration à la pression de réaction d'un circuit du réacteur.

12. Procédé selon la revendication 11, **caractérisé en ce que** le temps de séjour de la solution de réaction pendant la filtration est inférieur à 5 minutes.

13. Procédé selon les revendications 11 et 12, **caractérisé en ce que** la température de l'alimentation dans la filtration est abaissée dans une plage entre 5 et 40°C au-dessous de la température de réaction.

14. Procédé selon les revendications 11 à 13, **caractérisé en ce que** la filtration est réalisée en continu.

15. Procédé selon les revendications 1 à 14, **caractérisé en ce que** le mélange réactionnel est séparé en C₅-aldéhydes, gaz de synthèse, hydrocarbures et un résidu contenant du rhodium.
